Europäisches Patentamt

European Patent Office

Office européen des brevets

(11) Publication number: **0 405 902 A1**

# EUROPEAN PATENT APPLICATION

(21) Application number: 90306953.2

(22) Date of filing: 26.06.90

(51) Int. Cl.⁵: **C11D 3/386**, C12N 9/54

(30) Priority: 26.06.89 GB 8914605
07.07.89 GB 8915660

(43) Date of publication of application:
02.01.91 Bulletin 91/01

(84) Designated Contracting States:
CH DE ES FR GB IT LI NL SE

(71) Applicant: **UNILEVER PLC**
**Unilever House Blackfriars P.O. Box 68**
**London EC4P 4BQ(GB)**
(84) **GB**

Applicant: **UNILEVER NV**
**Burgemeester s'Jacobplein 1 P.O. Box 760**
**NL-3000 DK Rotterdam(NL)**
(84) **CH**

(72) Inventor: **Casteleijn, Eric**
**Palletierburg 105**
**NL-2907 CG Capelle a/d Ijssel(NL)**
Inventor: **Egmond, Maarten Robert**
**De Ness 34**
**NL-3461 GD Linschoten(NL)**
Inventor: **Haverkamp, Johan**
**De Kroeskarper 40**
**NL-2661 KL Bergschenhoek(NL)**
Inventor: **Marugg, John David**
**Fivelingo 141**
**NL-3524 BL Utrecht(NL)**
Inventor: **Mooren, Arnoldus Theodorus**
**Anthonius**
**Zwaluwenlaan 461**
**NL-3136 TP Vlaardingen(NL)**

(74) Representative: **Stancliffe, Terence**
**Christopher et al**
**UNILEVER PLC Patents Division P.O. Box 68**
**Unilever House**
**London EC4P 4BQ(GB)**

(54) **Enymatic detergent compositions.**

(57) Enzymatic detergent compositions comprise mutant subtilisin protease, in certain detergent compositions formulated as detergent powders containing phosphate or zeolite builder, aqueous detergent liquids, nonaqueous detergent liquids, or detergent bars.

## ENZYMATIC DETERGENT COMPOSITIONS

This invention relates to enzymatic detergent compositions, and in particular to compositions containing mutant subtilisin proteases. Prior-filed International Patent Application WO 89/06279 (not however prior-published) discloses certain mutant subtilisin-type proteases, and their use in washing compositions. The present invention relates in particular to novel detergent compositions incorporating mutant proteases as described in Application WO 89/06279.

In particular embodiments the invention further relates to the use of the modified proteases in conjunction with lipases.

Prior Art:

Subtilisin proteases and detergent compositions containing them, especially laundry detergents, are well known and have been widely used. Lipases are also known and used as ingredients of detergent and cleaning compositions.

A number of patent specifications and other documents describe various mutant hydrolytic enzymes including subtilisin proteases, some of which are expressed to be suitable for detergent or cleaning applications: Examples include EP 0 130 756 (Genentech), EP 0 214 435 (Henkel), WO 87/04461 (Amgen), WO 87/05050 (Genex), Ep 87303761 (Genentech), Ep 0 260 105 (Genencor), wO 88/08028 (Genex), WO 88/06624 (Gist-Brocades), WO 88/07578 (Genentech) and WO 88/08033 (Amgen). Thomas et al (in Nature, 318 (1985) 375-6) have described a mutation in subtilisin protease BPN' that changes the pH dependence of the enzyme.

Prior-filed International Patent Application WO 89/06279 (not however prior-published) discloses the mutant subtilisin-type proteases which are applied in the present application, and describes tests of them under certain washing conditions.

The present invention:

According to the present invention there is provided an enzymatic detergent composition, comprising a mutated subtilisin protease, characterised in that said protease contains, relative to the corresponding parent protease, an amino-acid residue at one or more of the following positions which has been changed by substitution, insertion or deletion:

6,9,11,12,19,25,36,37,38,53,54,55,56,57,58,59,67,71,89,
111,115,120,121,122,124,128,131,140,153,154,156,158,159,
160,161,162,163,164,165,166,168,170,172,175,180,182,186,
187,191,194,195,199,218,219,226,234,235,236,237,238,241, 260,261,262,265,268,275,

and in that the remainder of the detergent composition is either:

(a) formulated as a detergent powder containing phosphate builder, anionic surfactant, nonionic surfactant, acrylic or equivalent polymer, perborate bleach precursor, amino-containing bleach activator, silicate or other structurant, alkali to adjust to desired pH in use, and neutral inorganic salt; or

(b) formulated as a detergent powder containing zeolite builder, anionic surfactant, nonionic surfactant, acrylic or equivalent polymer, perborate bleach precursor, amino-containing bleach activator, silicate or other structurant, alkali to adjust to desired pH in use, and neutral inorganic salt; or

(c) formulated as an aqueous detergent liquid comprising anionic surfactant, nonionic surfactant, humectant, organic acid, caustic alkali, with a pH adjusted to a value between 9 and 10; or

(d) formulated as a nonaqueous detergent liquid comprising a liquid nonionic surfactant consisting essentially of linear alkoxylated primary alcohol, triacetin, sodium triphosphate, caustic alkali, perborate monohydrate bleach precursor, and tertiary amine bleach activator, with a pH adjusted to a value between about 9 and 10; or

(e) formulated as a detergent powder in the form of a granulate having a bulk density of at least 600 g/l, containing anionic surfactant and a mixture of nonionic surfactants with respective alkoxylation degrees about 7 and about 3, low or substantially zero neutral inorganic salt, phosphate builder, perborate bleach precursor, tertiary amine bleach activator, sodium silicate, and minors and moisture; or

(f) formulated as a detergent powder in the form of a granulate having a bulk density of at least 600 g/l, containing anionic surfactant and a mixture of nonionic surfactants with respective alkoxylation degrees about 7 and about 3, low or substantially zero neutral inorganic salt, zeolite builder, perborate bleach precursor, tertiary amine bleach activator, sodium silicate, and minors and moisture; or

(g) formulated as a detergent powder containing anionic surfactant, nonionic surfactant, acrylic polymer, fatty acid soap, sodium carbonate, sodium sulphate, clay particles, perborate bleach precursor, tertiary amine bleach activator, sodium silicate, and minors and moisture; or

(h) formulated as a detergent (soap) bar containing soap based on pan-saponified mixture of tallow and coconut oil, neutralised with orthophosphoric acid, mixed with protease, also mixed with sodium formate, borax, propylene glycol and sodium sulphate, and then plodded on a soap production line.

Detergent compositions according to the present invention can give advantages including improved performance and/or enzyme stability as compared with known compositions.

References herein to numbered aminoacid sequence positions which are the subject of mutations in subtilisin proteases to be used in accordance with this invention, are references to aminoacid residues and their number as they occur in the following sequence, which is that of subtilisin BPN'. The invention also includes modified variants of other subtilisin proteases, and in order to apply the numbered mutation sites referred to herein to such another protease, the numerical part of the reference is to be understood as a reference to the corresponding position of such other homologous subtilisin protease in the sense of its maximum homology with subtilisin BPN'. Such a corresponding position may differ in number along the chain of the other protease by reason of apparent deletion(s) or insertion(s) in the gene of such other protease by comparison with that of the gene of BPN' or other reference sequence. A deletion or absent aminoacid is indicated by '*', and an insertion relative to BPN' by a lower case alphabetical suffix on the position number.

Subtilisin BPN' has the following sequence (ref Wells et al (1983) Nucleic Acids Res. 11, 7911-7925):

```
No:              1                    10
    *-*-*-*-*-*-*-A-Q-S-*-V-P-Y-G-V-S-Q-I-K-*-*-*-*-*-A-P-A-
No:      20                  30                   40
    L-H-S-Q-G-Y-T-G-S-N-V-K-V-A-V-I-D-S-G-I-D-S-S-H-P-D-L-*-
No:                      50                  60
    *-K-V-A-G-G-A-S-M-V-P-S-E-T-N-P-F-*-*-Q-D-N-N-S-H-G-T-H-V-
No:  70                  80                   90
    A-G-T-V-A-A-L-*-N-N-S-I-G-V-L-G-V-A-P-S-A-S-L-Y-A-V-K-V-
No:      100                 110                     120
    L-G-A-D-G-S-G-Q-Y-S-W-I-I-N-G-I-E-W-*-A-I-A-*-N-N-M-D-*-
No:                          130                 140
```

EP 0 405 902 A1

*-*-*-*-V-I-N-M-S-L-G-G-P-S-G-S-A-A-L-K-A-A-V-D-K-A-V-A-
No:             150                 160                 170
S-G-V-V-V-V-A-A-G-N-E-G-T-S-G-S-S-S-T-V-G-Y-P-G-K-Y-P-
No:             180                 190                 200
S-V-I-A-V-G-A-V-D-S-S-N-Q-R-A-S-F-S-S-V-G-P-E-L-D-V-M-A-
No:                   210                 220
P-G-V-S-I-Q-S-T-L-P-G-N-*-K-*-Y-G-A-Y-N-G-T-S-M-A-S-P-H-
No:       230                 240                 250
V-A-G-A-A-A-L-I-L-S-K-H-P-N-W-T-N-T-Q-V-R-S-S-L-E-N-T-T-
No:             260                 270       275
T-K-L-G-D-S-F-Y-Y-*-G-K-G-L-I-N-V-Q-A-A-A-Q

Subtilisin 309 has the following sequence:
(ref Patent Application PCT/DK 88/00002=WO 89/06279)

No:                         1                 10
*-*-*-*-*-*-*-A-Q-S-*-V-P-W-G-I-S-R-V-Q-*-*-*-*-*-A-P-A-
No:       20                  30                  40
A-H-N-R-G-L-T-G-S-G-V-K-V-A-V-L-D-T-G-I-*-S-T-H-P-D-L-*-
No:             50                  60
*-N-I-R-G-G-A-S-F-V-P-G-E-P-*-S-T-*-*-Q-D-G-N-G-H-G-T-H-V-
No:  70                  80                  90
A-G-T-I-A-A-L-*-N-N-S-I-G-V-L-G-V-A-P-S-A-E-L-Y-A-V-K-V-
No:       100                 110                 120
L-G-A-S-G-S-G-S-V-S-S-I-A-Q-G-L-E-W-*-A-G-N-*-N-G-M-H-*-
No:                   130                 140
*-*-*-*-V-A-N-L-S-L-G-S-P-S-P-S-A-T-L-E-Q-A-V-N-S-A-T-S-
No:             150                 160                 170
R-G-V-L-V-V-A-A-S-G-N-S-G-A-*-G-S-I-S-*-*-*-Y-P-A-R-Y-A-
No:             180                 190                 200
N-A-M-A-V-G-A-T-D-Q-N-N-N-R-A-S-F-S-Q-Y-G-A-G-L-D-I-V-A-
No:                   210                 220
P-G-V-N-V-Q-S-T-Y-P-G-S-*-T-*-Y-A-S-L-N-G-T-S-M-A-T-P-H-
No:       230                 240                 250

V-A-G-A-A-A-L-V-K-Q-K-N-P-S-W-S-N-V-Q-I-R-N-H-L-K-N-T-A-
No:             260                 270       275
T-S-L-G-S-T-N-L-Y-*-G-S-G-L-V-N-A-E-A-A-T-R

In the above sequences the individual letter codes indicate amino-acid residues in accordance with the

4

following abbreviations:-

A = Ala = Alanine
V = Val = Valine
L = Leu = Leucine
I = Ile = Isoleucine
P = Pro = Proline
F = Phe = Phenylalanine
W = Trp = Tryptophan
M = Met = Methionine
G = Gly = Glycine
S = Ser = Serine
T = Thr = Threonine
C = Cys = Cysteine
Y = Tyr = Tyrosine
N = Asn = Asparagine
Q = Gln = Glutamine
D = Asp = Aspartic acid
E = Glu = Glutamic acid
K = Lys = Lysine
R = Arg = Arginine
H = His = Histidine

It has also been surprisingly found that compatibility of protease with lipase is influenced by the pl of the protease and by the positions at which the charges are located relative to the active site of the protease: the introduction of negative charge or removal of positive charge to give a decrease in the isoelectric point pl, can improve compatibility of protease with lipase, and making the mutations of this kind closer to the active site can give stronger improvement of compatibility of protease with lipase.

The isoelectric point, pl, is the pH value where the enzyme molecule (with optionally attached metal or other ions) is neutral, i. e. the sum of electrostatic charges is zero.

The isoelectric point is conveniently calculated (approximately) by using pK values for the various charged residues in the enzyme in question and then by iteration find the pH value where the net charge of the enzyme is zero.

The pl can also be determined experimentally by isoelectric focussing or by titrating a solution containing the enzyme. The various pK values for the charged residues may also be determined by titration.

(Mutant proteases with modified pl are described in copending application    (claiming priority from UK 8914604.7 of 26/6/89), which describes and claims detergent compositions containing them, and the corresponding mutant proteases themselves are described and claimed in copending application (claiming priority from DK 3169/89 of 26/6/89).)

In certain embodiments of the detergent compositions the mutated subtilisin protease contains, relative to the corresponding parent protease, an amino-acid residue at within about 20A of the active site which has been changed by substitution, deletion, or adjacent insertion, e.g. at one or more of positions 6, 36-38, 53-59, 89, 128, 131, 156, 158-160, 162-164, 166, 170, 182, 186, 195, and/or 218.

Detailed information about the structure and aminoacid numbering sequence of the subtilisin proteases, are given in prior-filed International Patent Application WO 89/06279, and in copending application (claiming priority from UK Patent Application 8914604.7 of 26/6/89), both incorporated herein by reference.

Techniques for the production of the mutant proteases are described in WO 89/06279 (hereby incorporated herein by reference), and further applicable such methods are also given in the above-cited copending application (also incorporated herein by reference).

In particular detergent compositions within the scope of the invention, the protease can represent a mutation of a parent enzyme selected from subtilisin BPN', subtilisin amylo-saccharolyticus, subtilisin 168, subtilisin mesinticopeptidase, subtilisin Carlsberg, subtilisin DY, subtilisin 309, subtilisin 147, subtilisin thermitase, and proteinase K, or aqualysin.

In particular examples, the protease can represent a mutation of a parent enzyme altered to include any of the following amino-acid residues indicated by their abbreviations and located at the indicated sequence position: any of the mutations 6-tyr, 67-glu, 67-asp, 68-cys, 68-met, 71-glu, 168-ala, 170-tyr, 175-ile, 219-met, 275-glu, 19-gly + 219-cys, 153-ala + 218-ser.

In further examples, the protease mutation can consist of an insertion of one or more aminoacid residues at any of positions 36,56,159,164-166.

Specific mutant examples preferred for use in connection with this invention are as follows and referred to by the following labels:

S1 = G195E;

S3 = R170Y;

S4 = G195E + R170Y;

S12 = G195E + R170Y + K251E;

S19 = G195E + R170Y + H120D + K235L;

S20 = G195E + R170Y + H120D + K235L + K251E;

C2 = D120K;

C3 = D140K;

C4 = D120K + D14K;

C6 = D120K + K27D;

C8 = D172K;

C10 = D14K + D120K + D140K + D172K.

The S series mutants are preferably based on subtilisin 309 as the parent subtilisin. The C mutants are preferably based on subtilisin BPN'/subtilisin Carlsberg.

In particular embodiments the invention presents enzymatic detergents comprising the proteases defined herein and with the features:

An enzymatic detergent composition formulated to give a wash liquor pH of 9 or less when used at a rate corresponding to 0.4-0.8 g/l surfactant.

An enzymatic detergent composition formulated to give a wash liquor pH of 8.5 or more when used at a rate corresponding to 0.4-0.8 g/l surfactant.

An enzymatic detergent composition formulated to give a wash liquor ionic strength of 0.03 or less, e.g. 0.02 or less, e.g. 0.01 or less, when used at a rate corresponding to 0.4-0.8 g/l surfactant.

An enzymatic detergent composition formulated to give a wash liquor ionic strength of 0.01 or more, e.g. 0.02 or more, when used at a rate corresponding to 0.4-0.8 g/l surfactant.

The invention is illustrated by way of the following non-limitative Examples:

Example 1:

A detergent powder according to an embodiment of the invention containing phosphate builder is formulated to contain: total active detergent about 16%, anionic detergent about 9%, nonionic detergent about 6%, phosphate-containing builder about 20%, acrylic or equivalent polymer about 3.5%, perborate bleach precursor about 6-18%, amino-containing bleach activator about 2%, silicate or other structurant about 3.5%, enzyme about 8 glycine units/mg, with alkali to adjust to desired pH in use, and neutral inorganic salt, and enzymes (about 0.5% each enzyme).

The anionic detergent is a mixture of sodium dodecylbenzene sulphonate 6% and primary alkyl sulphate 3%. The nonionic detergent is an ethoxylate of an approx. C13-C15 primary alcohol with 7 ethoxylate residues per mole. The phosphate builder is sodium tripolyphosphate. The polymer is polyacrylic acid. The perborate bleach precursor is sodium tetraborate tetrahydrate or monohydrate. The activator is tetraacetylethylenediamine. The structurant is sodium silicate. The neutral inorganic salt is sodium sulphate. The enzyme is a protease according to Example S1. In alternative versions of this Example, the enzyme is selected from S3, S4, C2, C3, or C4.

Example 2:

A detergent powder according to an embodiment of the invention containing zeolite builder is formulated to contain: total active detergent about 16%, anionic detergent about 9%, nonionic detergent about 6%, zeolite-containing builder about 20%, acrylic or equivalent polymer about 3.5%, perborate bleach precursor about 6-18%, amino-containing bleach activator about 2%, silicate or other structurant about 3.5%, enzyme about 8 glycine units/mg, with alkali to adjust to desired pH in use, and neutral inorganic salt, and enzymes (about 0.5% each enzyme).

The anionic detergent is a mixture of sodium dodecylbenzene sulphonate 6% and primary alkyl sulphate 3%. The nonionic detergent is an ethoxylate of an approx. C13-C15 primary alcohol with 7 ethoxylate residues per mole. The zeolite builder is type A zeolite. The polymer is polyacrylic acid. The perborate bleach precursor is sodium tetraborate tetrahydrate or monohydrate. The activator is tetraacetylethylenediamine. The structurant is sodium silicate. The neutral inorganic salt is sodium sulphate. The enzyme is a protease according to Example S1. In alternative versions of this Example, the enzyme is selected from S3, S4, C2, C3, or C4.

Example 3:

An aqueous detergent liquid according to an embodiment of the invention is formulated to contain: Dodecylbenzene-sulphonic acid 16%, C12-C15 linear alcohol condensed with 7 mol/mol ethylene oxide 7%, monoethanolamine 2%, citric acid 6.5%, sodium xylenesulphonate 6%, sodium hydroxide about 4.1%, protease 0.5%, minors and water to 100%. The pH is adjusted to a value between 9 and 10. The enzyme is a protease according to Example S20. In alternative versions of this Example, the enzyme is selected from S19, S12, S1, S3 or S4.

Example 4:

A nonaqueous detergent liquid according to an embodiment of the invention is formulated using 38.5% C13-C15 linear primary alcohol alkoxylated with 4.9 mol/mol ethylene oxide and 2.7 mol/mol propylene oxide, 5% triacetin, 30% sodium triphosphate, 4% soda ash, 15.5% sodium perborate monhydrate containing a minor proportion of oxoborate, 4% TAED, 0.25% EDTA of which 0.1% as phosphonic acid, Aerosil 0.6%, SCMC 1%, and 0.6% protease. The pH is adjusted to a value between 9 and 10, e.g. about 9.8. The enzyme is a protease according to Example S1, S3 or S4.

Example 5:

A detergent powder according to an embodiment of the invention is formulated in the form of a granulate having a bulk density of at least 600 g/l, containing about 20% by weight surfactant of which about 10% is sodium dodecylbenzene sulphonate, and the remainder is a mixture of Synperonic A7 and Synperonic A3 (about 5.5% to 4.5%), and zero neutral inorganic salt (e.g. sodium sulphate), plus phosphate builder about 33%, sodium perborate tetrahydrate about 16%, TAED activator about 4.5%, sodium silicate about 6%, and minors including sodium carbonate about 2%, and moisture content about 10%. Enzymes (about 0.5% each enzyme) are included. The protease enzyme is a protease according to Example S1. In alternative versions of this Example, the enzyme is selected from S3, S4, C2, C3, or C4.

Example 6:

A detergent powder according to an embodiment of the invention is formulated in the form of a granulate having a bulk density of at least 600 g/l, containing about 20% by weight surfactant of which about 9% is sodium dodecylbenzene sulphonate, and the remainder is a mixture of Synperonic A7 and Synperonic A3 (respectively about 5% & 6%), and zero neutral inorganic salt (e.g. sodium sulphate), plus zeolite builder about 30%, sodium perborate tetrahydrate about 14%, TAED activator about 3.6%, and minors including sodium carbonate about 9%, Dequest 2047 (TM) about 0.7%, and moisture content about 10%. Enzymes (about 0.5% each enzyme) are included. The protease enzyme is a protease according to Example S1. In alternative versions of this Example, the enzyme is selected from S3, S4, C2, C3, or C4.

Example 7:

A detergent powder according to an embodiment of the invention is formulated to contain: Dodecylbenzenesulphonic acid 6%, C12-C15 linear alcohol condensed with 7 mol/mol ethylene oxide 5%, fatty acid soap 3%, Sokolan CP5 polymer (TM) 3%, zeolite A 22%, sodium carbonate 10%, sodium sulphate 17%, clay particles 8%, sodium perborate tetrahydrate 13%, tetraacetylethylenediamine 2%, protease 0.5%, minors and water to 100%. The pH is adjusted to a value between 9 and 10. The enzyme is a protease according to Example S20. In alternative versions of this Example, the enzyme is selected from S19, S12, S4, S1, or S3.

7

Example 8:

A detergent (soap) bar according to an embodiment of the invention is formulated as follows: soap based on pansaponified 82% tallow, 18% coconut oil, neutralised with 0.15% orthophosphoric acid, mixed with protease (about 8 GU/mg of the bar composition) and mixed with sodium formate 2%, borax 2%, propylene glycol 2% and sodium sulphate 1%, is then plodded on a soap production line. The protease enzyme is a protease according to Example S1. In alternative versions of this Example, the enzyme is selected from S3, S4, C2, C3, or C4.

In further embodiments of the invention, structured liquid detergents can for example contain, in addition to a protease as described herein, 2-15% nonionic surfactant, 5-40% total surfactant, comprising nonionic and optionally anionic surfactant, 5-35% phosphate-containing or non-phosphate-containing builder, 0.2-0.8% polymeric thickener, e.g. cross-linked acrylic polymer with m.w. over $10^6$, at least 10% sodium silicate, e.g. as neutral waterglass, alkali (e.g. potassium-containing alkali) to adjust to desired pH, preferably in the range 9-10 or upwards, e.g. above pH 11, with a ratio sodium cation: silicate anion (as free silica) (by weight) less than 0.7:1, and viscosity of 0.3-30 Pa.s (at 20 deg. C and 20 reciprocal secs).

For example such detergents can contain about 5% nonionic surfactant C13-15 alcohol alkoxylated with about 5 EO groups per mole and with about 2.7 PO groups per mole, 15-23% neutral waterglass with 3.5 weight ratio between silica and sodium oxide, 13-19% KOH, 8-23% STPP, 0-11% sodium carbonate, 0.5% Carbopol 941 (TM). Protease may be incorporated at for example 0.5% of example S1.

The invention is susceptible of modifications and variations of the present disclosure which extends to all combinations and subcombinations of the features mentioned and described herein, in the following claims and in the specifications referred to herein and incorporated by reference.

## Claims

1: An enzymatic detergent composition, comprising a mutated subtilisin protease, characterised in that said protease contains, relative to the corresponding parent protease, an amino-acid residue at one or more of the following positions which has been changed by substitution, insertion or deletion:
6,9,11,12,19,25,36,37,38,53,54,55,56,57,58,59,67,71,89,
111,115,120,121,122,124,128,131,140,153,154,156,158,159,
160,161,162,163,164,165,166,168,170,172,175,180,182,186,
187,191,194,195,199,218,219,226,234,235,236,237,238,241, 260,261,262,265,268,275,
and in that the remainder of the detergent composition is either:

(a) a detergent powder containing phosphate builder, anionic surfactant, nonionic surfactant, acrylic or equivalent polymer, perborate bleach precursor, amino-containing bleach activator, silicate or other structurant, alkali to adjust to desired pH in use, and neutral inorganic salt; or

(b) a detergent powder containing zeolite builder, anionic surfactant, nonionic surfactant, acrylic or equivalent polymer, perborate bleach precursor, amino-containing bleach activator, silicate or other structurant, alkali to adjust to desired pH in use, and neutral inorganic salt; or

(c) an aqueous detergent liquid comprising anionic surfactant, nonionic surfactant, humectant, organic acid, caustic alkali, with a pH adjusted to a value between 9 and 10; or

(d) a nonaqueous detergent liquid comprising a liquid nonionic surfactant consisting essentially of linear alkoxylated primary alcohol, triacetin, sodium triphosphate, caustic alkali, perborate monohydrate bleach precursor, and tertiary amine bleach activator, with a pH adjusted to a value between about 9 and 10; or

(e) a detergent powder in the form of a granulate having a bulk density of at least 600 g/l, containing anionic surfactant and a mixture of nonionic surfactants with respective alkoxylation degrees about 7 and about 3, low or substantially zero neutral inorganic salt, phosphate builder, perborate bleach precursor, tertiary amine bleach activator, sodium silicate, and minors and moisture; or

(f) a detergent powder in the form of a granulate having a bulk density of at least 600 g/l, containing anionic surfactant and a mixture of nonionic surfactants with respective alkoxylation degrees about 7 and about 3, low or substantially zero neutral inorganic salt, zeolite builder, perborate bleach precursor, tertiary amine bleach activator, sodium silicate, and minors and moisture; or

(g) a detergent powder containing anionic surfactant, nonionic surfactant, acrylic polymer, fatty acid soap, sodium carbonate, sodium sulphate, clay particles, perborate bleach precursor, tertiary amine bleach activator, sodium silicate, and minors and moisture; or

(h) a detergent (soap) bar containing soap based on pansaponified mixture of tallow and coconut oil, neutralised with orthophosphoric acid, mixed with protease, also mixed with sodium formate, borax, propylene glycol and sodium sulphate, and then plodded on a soap production line.

2: A detergent composition as claimed in claim 1, further characterised in that the protease represents a mutation of a parent enzyme selected from subtilisin BPN', subtilisin amylosaccharolyticus, subtilisin 168, subtilisin mesinticopeptidase, subtilisin Carlsberg, subtilisin DY, subtilisin 309, subtilisin 147, subtilisin thermitase, and proteinase K or aqualysin.

3: A detergent composition as claimed in any preceding claim, further characterised in that the protease is based on subtilisin 309 as the parent subtilisin.

4: A detergent composition as claimed in any preceding claim, further characterised in that the protease is based on subtilisin 147 as the parent subtilisin.

5: A detergent composition as claimed in any preceding claim, further characterised in that the protease contains one or more of the following mutations:

(a) insertion of one or more aminoacid residues at any of positions 36,56,159,164-166; or

(b) any of the mutations 6-tyr, 67-glu, 67-asp, 68-cys, 68-met, 71-glu, 168-ala, 170-tyr, 175-ile, 219-met, 275 glu, 19-gly + 219-cys, 153-ala + 218-ser.

6: An enzymatic detergent composition, as claimed in claim 1, characterised in that the detergent composition is formulated to give a wash liquor pH of 9 or less when used at a rate corresponding to 0.4-0.8 g/l surfactant.

7: An enzymatic detergent composition, as claimed in claim 1, characterised in that the detergent composition is formulated to give a wash liquor ionic strength of 0.03 or less, e.g. 0.02 or less, e.g. 0.01 or less, when used at a rate corresponding to 0.4-0.8 g/l surfactant.

8: An enzymatic detergent composition, as claimed in claim 1, characterised in that the detergent composition is formulated to give a wash liquor pH of 8.5 or more when used at a rate corresponding to 0.4-0.8 g/l surfactant.

9: An enzymatic detergent composition, as claimed in claim 1, characterised in that the detergent composition is formulated to give a wash liquor ionic strength of 0.01 or more, e.g. 0.02 or more, when used at a rate corresponding to 0.4-0.8 g/l surfactant.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 251 446 (GENENTECH INC.) <br> * Claims; page 24, table 1 * | 1-9 | C 11 D 3/386 <br> C 12 N 9/54 |
| D,Y | WO-A-8 808 028 (GENEX CORP.) <br> * Claims; page 21, line 10 - page 22, line 12 * | 1-9 | |
| D,P <br> Y | WO-A-8 906 279 (NOVO INDUSTRI A/S) <br> * Claims; page 10, lines 16-27; page 42, lines 20-28 * | 1-9 | |
| D,Y | NATURE, vol. 328, August 1987, pages 496-500, London, GB; A.J. RUSSELL et al.: "Rational modification of enzyme catalysis by engineering surface charge" <br> * Whole article * | 1-9 | |

TECHNICAL FIELDS SEARCHED (Int. Cl.5)

C 12 N
C 11 D

The present search report has been drawn up for all claims

| Place of search | Date of completion of the search | Examiner |
|---|---|---|
| THE HAGUE | 24-09-1990 | HUBER A. |